(19) Europäisches Patentamt
European Patent Office
Office européen des brevets

(11) Numéro de publication: **0 244 318**
**B1**

# (12) FASCICULE DE BREVET EUROPEEN

(45) Date de publication du fascicule du brevet: **05.09.90**

(51) Int. Cl.$^5$: **C 07 D 211/22,**
C 07 D 211/60, A 61 K 31/445

(21) Numéro de dépôt: **87400970.7**

(22) Date de dépôt: **27.04.87**

## (54) Nouveaux dérivés de la N-(triméthoxy-2,4,6-benzoyl)-3 propylr pipéridine, leurs procédés de préparation et leur application en thérapeutique.

(30) Priorité: **28.04.86 FR 8606133**

(43) Date de publication de la demande:
**04.11.87 Bulletin 87/45**

(45) Mention de la délivrance du brevet:
**05.09.90 Bulletin 90/36**

(84) Etats contractants désignés:
**AT BE CH DE ES FR GB GR IT LI LU NL SE**

(56) Documents cités:
**EP-A-0 063 075**
**FR-A-2 101 045**
**FR-A-2 534 912**

(73) Titulaire: **LABORATOIRE L. LAFON**
**19 Avenue du Professeur Cadiot**
**F-94701 Maisons Alfort (FR)**

(72) Inventeur: **Lafon, Louis**
**5 Rue de l'Alboni**
**F-75016 Paris (FR)**

(74) Mandataire: **Moncheny, Michel et al**
**c/o Cabinet Lavoix 2 Place d'Estienne d'Orves**
**F-75441 Paris Cedex 09 (FR)**

EP 0 244 318 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La presente invention concerne de nouveaux dérivés de la N-[(triméthoxy-2,4,6-benzoyl)-3 propyl] pipéridine, leurs procédés de préparation et leur application en thérapeutique.

On a déjà décrit un certain nombre de dérivés de la N-[(triméthoxy-2,4,6-benzoyl)-3 propyl] pipéridine ou de composés voisins:

Le brevet US—A—3 895 030 décrit en particulier la (triméthoxy-2,4,6-phénol) (pyrrolidino-3 propyl)cétone (Buflomédil) qui est un agent vasodilateur périphérique commercialisé sous le nom de Fonzylane et la (triméthoxy-2,4,6 phényl) (pipéridino-3 propyl)cétone, qui présente une activité antispasmodique.

Le brevet FR—A—2 404 003 décrit des (hydroxyphényl) (pyrrolidino-3 propyl)cétones qui possèdent une activité vasodilatatrice.

Le brevet FR—A—2 534 912 décrit des dérivés de (triméthoxy-2,4,6 phényl) (pipéridino-3 propyl)cétone et notamment la triméthoxy-2,4,6 phényl)-[(méthyl-3 pipéridino)-3 propyl]cétone (CRL 41034) qui possède des propriétés vasodilatatrices et hyoptensives.

Le brevet GB—A—1 115 992 decrit la N(methyl-4'-pipéridino) triméthoxy-2,4,6 acétophénone qui presente une activité antispasmodique, tranquilisante et analgésique.

Le brevet FR—A—2 101 045 décrit des [(triméthoxy-2,4,6-benzoyl)-3 propyl] amines et en particulier la [(triméthoxy-2,4,6-benzoyl)-3 propyl] pipéridine qui possèdant des propriétés vasodilatatrices et antispasmodique.

Dans le brevet EP—A—0 063 075 on a en outre décrit la (2,6-diméthoxy-4-hydroxyphényl)-(3-pipéridinopropyl) cétone qui possède des propriétés vasodilatatrices périphériques.

La Demanderesse a trouvé une nouvelle classe de dérivés de la N (triméthoxy-2,4,6-benzoyl)-3 propyl pipéridine qui présentent des activités pharmacologiques originales utilisables en thérapeutique.

La présente invention a ainsi pour objet des composés de formule

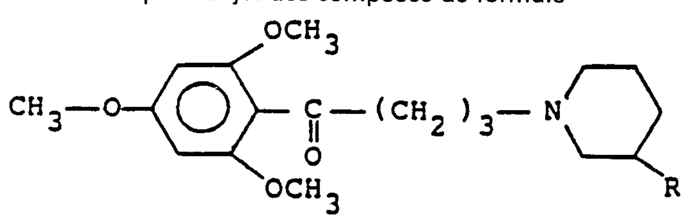

I

dans laquelle R est choisi parmi les groupes $—CH_2OH$, $—COOH$, et $—COOR_1$, $R_1$ etant un groupe alkyle en $C_1$ à $C_4$,

et leurs sels pharmaceutiquement acceptables.

La présente invention vise plus particulièrement le N-[(triméthoxy-2,4,6-benzoyl)-3 propyl] pipéridine-3-méthanol et ses sels pharmacetiquement acceptables qui se caractérisent par une activité antiarythmisante.

La présente invention a également pour objet des compositions thérapeutiques contenant, à titre de principe actif, un composé de formule (I) ou l'un de ses sels pharmaceutiquement acceptables.

Par sels pharmaceutiquement acceptables, on désigne les sels d'addition que forment les composés de formule (I) avec des acides pharmaceutiquement acceptables, ainsi que les sels que forment le composé de formule (I) à groupe acide avec des bases pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide lactique, l'acide succinique, l'acide tartrique et l'acide pamoïque.

De même, "les sels avec des bases pharmaceutiquement acceptables" désignent les sels qui ne modifient pas les propriétés biologiques des acides libres. Ces sels peuvent être notamment ceux formés avec des bases minérales, telles que l'hydroxyde de sodium, l'hydroxyde de potassium, l'hydroxyde de lithium, l'hydroxyde de calcium, l'hydroxyde de magnésium, ou des bases organiques telles que la glucamine, le N-méthyl-glucamine, la N,N-diméthylglucamine, l'éthanolamine, la diéthanolamine, la morpholine, la N-méthyl morpholine, la tris-(hydroxy-méthyl)-méthylamine et la lysine.

Les composés selon la présente invention peuvent être obtenus par condensation d'un composé de formule

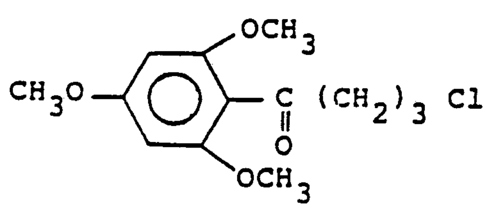

II

avec une pipéridine de formule

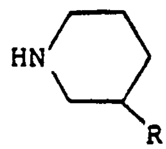

III

Cette réaction peut être effectuée dans un solvant organique tel que le toluène en présence d'un excès de composé de formule III.

Le composé de formule II peut être obtenu par une réaction de Friedel et Crafts entre l'éther triméthylique du phloroglucinol et le chlorure d'ω-chlorobutyryle.

En variante on peut obtenir les composés de formule I par condensation d'un butyronitrile de formule

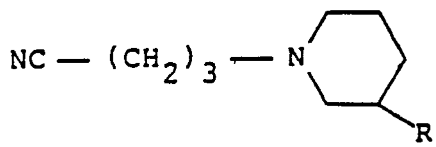

IV

sur l'éther triméthylique du phloroglucinol, selon une réaction de Houben-Hoesch, en présence d'acide chlorhydrique sans catalyseur, et hydrolyse acide subséquente.

Les butyronitriles de formule IV peuvent être obtenus par condensation du chloro-4-butyronitrile sur une pipéridine de formule III dans un solvant tel que le toluène.

Les exemples suivants illustrent la préparation des composés de formule I.

Exemple 1

Préparation du N-[(triméthoxy-2,4,6-benzoyl)-3 propyl] pipéridiné-3-méthanol, chlorhydrate (n° de code CRL 41 391)

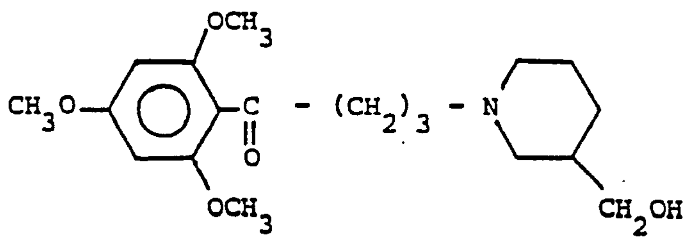

, HCl

a) Préparation du(triméthoxy-2,4,6 benzoyl)-1-chloro-3 propane

Au sein d'une solution maintenue vers 5°C de 33,6 g (0,200 mole) de triméthoxy-1,3,5 benzène et de 30,5 g (0,216 mole) de chlorure de chloro-4 butyryle dans 150 ml de benzène, on coule en 1 h 30 une solution de 60,5 g (0,232 mole) de chlorure stannique dans 75 ml de benzène et on agite 3 h à la température ambiante. On jette le milieu réactionnel sur 250 ml d'acide chlorhydrique 4 N glacé et on décante la phase organique.

Après séchage sur sulfate de sodium sec et évaporation du solvant, on obtient: 55 g d'une huile orangée. Rendement 100%.

b) Préparation du N-[(triméthoxy-2,4,6 benzoyl)-3-propyl]pipéridine-3-méthanol, chlorhydrate

Au sein d'une solution au reflux de 23 g (0,20 mole) de pipéridine-3 méthanol dans 60 ml de toluène, on coule en 1 h une solution de 27,25 g (0,10 mole) du produit obtenu selon a) dans 20 ml de toluène, et on maintient le reflux 1 h 30. On lave le milieu réactionnel par de l'eau et on l'extrait par de l'acide chlorhydrique N. Après alcalinisation de la phase aqueuse par de la soude 4 N on isole 27,8 g d'une huile orangée.

Cette huile est traitée dans l'éther éthylique par de l'éthanol chlorhydrique et le précipité obtenu, purifié par une cristallisation dans le mélange acétonitrile/isopropanol (9/1), pour donner: 20 g d'une poudre légèrement beige soluble dans l'eau. F. inst. (Kopfler) = 177°C. Rendement = 51,6%.

Exemple 2

Préparation du N-[(triméthoxy-2,4,6-benzoyl)-3 propyl] nipécotate d'éthyle, chlorhydrate (n° de code CRL 41 389)

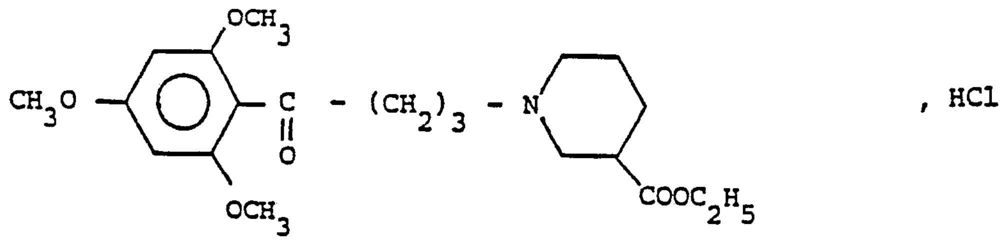

Au sein d'une solution au reflux de 31,4 ml (0,20 mole) de nipécotate d'éthyle dans 60 ml de toluène, on coule en 1 h, une solution de 27,25 g (0,10 mole) du produit obtenu à l'exemple 1a dans 20 ml de toluene et on poursuit le reflux 3 heures. On lave le milieu réactionnel par de l'eau et on l'etrait par une solution d'acide chlorhydrique N. Après alcalinisation par du carbonate de sodium, la phase aqueuse libère: 31,4 g d'une huile légèrement verte.

Cette huile est traitée dans l'éther éthylique par de l'éthanol chlorhydrique et le précipité obtenu purifié par deux cristallisations successives avec traitement au noir 3 S dans le mélange acétate d'éthyle/éthanol (3/1) et l'acétonitrile pour donner: 12 g d'une poudre blanche soluble dans l'eau. F. inst. (Kopfler) = 155°C. Rendemenet = 27,9%.

Exemple 3

Préparation de l'acide N-[(triméthoxy-2,4,6-benzoyl)-3 propyl] pipéridiné-3 carboxylique, chlorhydrate (n° de code CRL 41 390)

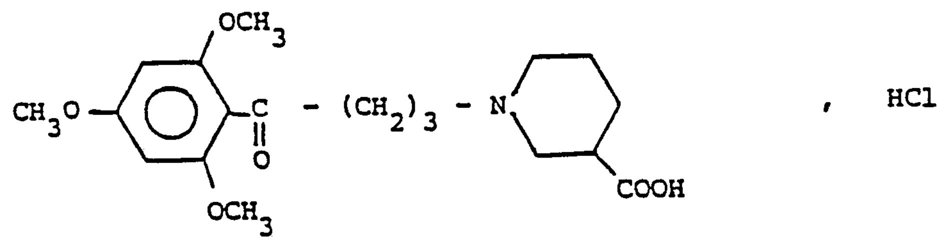

a) Préparation du N-(cyano-3 propyl) nipécotate d'éthyle

Au sein d'une solution au reflux de 100 g (0,636 mole) de nipécotate d'éthyle dans 80 ml de toluène et en présence d'une trace d'iodure de potassium, on coule en 1 h: 32,9 g (0,318 mole) de chloro-4 butyronitrile et on maintient le reflux encore 2 heures. On dilue le milieu réactionnel par de l'éther éthylique, on élimine le précipité par filtration et on lave le filtrat par de l'eau.

Après séchage et évaporation du solvant, on purifie le résidu par une distillation sous pression réduite pour donner: 50,6 g d'une huile incolore. $E_{5mm}$ = 152—154°C. Rendement = 71,1%.

b) Préparation de l'acide N-[(triméthoxy-2,4,6-benzoyl)-3 propyl] pipéridine-3-carboxylique chlorhydrate

On sature en 3 heures, vers 0°C, par un courant d'acide chlorhydrique, une solution de 16,8 g (0,10 mole) de triméthoxy-1,3,5 benzène et 22,4 g (0,10 mole) du produit obtenu en a) dans 120 ml de chlorobenzène anhydre. On extrait la masse gélatineuse rose par de l'eau, on porte la phase aqueuse au reflux 1 heure et on l'amène à siccité sous pression réduite. Le résidu est séché par distillation azéotropique dans du benzène et le précipité obtenu isolé par filtration.

Ce produit est purifié par deux cristillisations successives dans l'éthanol absolu puis dans le mélange acétonitrile/méthanol (9/1) pour donner: 13 g d'une poudre blanche soluble dans l'eau et dans la soude. F.≃190°C. Rendement = 32,4%. Rendement total = 23%.

On donnera ci-après des résultats des études pharmacologiques.

1. Composé de l'exemple 1 (CRL 41391)
a) Toxicité aigüe

| Dose I.P. mg/kg | Mortalité /6 souris |
|---|---|
| 195 | 0 |
| 232.5 | $3^{+}$ |
| 260 | $5^{+}$ |
| $DL_{50}$/232 mg/kg | |

$^{+}$ convulsions

b) Action sur le système cardiovasculaire (chez le chien)

3 chiens (poids moyen: 12 kg) anesthésiés au nembutal reçoivent le composé par voie intraduodénale, aux doses successives de 0,1—0,5—2,5—10 et 20 mg/kg.

On mesure la pression artérielle, la fréquence cardiaque, le débit artérielle fémoral, le débit artériel vertébral, les températures rectale et cutanée. On observer la coloration de la peau.

Le composé n'a pas d'effet sur la pression artérielle. Il augmente le débit fémoral à partir de la dose de 5 mg/kg et diminue le débit vertébral. Il est légèrement bradycardisant à 20 mg/kg. Il ne modifie pas les températures rectals et cutanée.

Les effets de l'isoprénaline testés après la dose cumulée de 39,1 mg/kg du composé I.D. sont très légèrement diminués sur la pression artérielle et sur la fréquence cardiaque: à 10 mcg/kg par voie intravenieuse d'isoprénaline, la pression artérielle diastolique passe après produit de 107 à 36 mmHg ( 71)/ 120 à 37 mmHg ( 83) en témoin, et la fréquence cardiaque passe après produit de 160 à 255 bat/min/157 à 273 bat/min en témoin. L'hypertension à la noradrénaline est légèrement diminuée: à 2 mcg/kg par voie intraveineuse de noradrènaline, la pression artérielle systolique passe après produit de 149 à 256 mmHg/ 153 à 271 mmHg en témoin.

Deux de ces trois chiens ayant subi une ligature d'une artère coronaire quelques semaines auparavant présentent encore de nombreux battements ectopiques. Le composé a rétabli un rythme normal chez ces deux chiens à 2,5 mg/kg I.D. pour l'un et 10 mg/kg I.D. pour l'autre.

Chez le troisième chien, des arythmies ont été déclenchées par injections d'adrénaline (10 mcg/kg I.V.) précédée d'une injection intra-trachéale d'éther de pétrole (0,1 ml/kg) avant et après administration des 39,1 mg/kg (en doses cumulées) du composé. On compte sur l'E.C.G. le nombre de battements d'origine sinusale et le nombre de battements ectopiques dans la minute qui suit l'injection d'adrénaline.

| Dose de composé mg/kg I.D. | Nombre de battements en 1 minute | |
|---|---|---|
| | ectopiques | sinusaux |
| 0 | 163 | 35 |
| 39,1 | 26 | 173 |

Le composé diminue fortement le nombre de battements ectopiques entraînés par adrénaline + éther de pétrole.

Chez un chien dont on a ligaturé 24 h plus tôt l'artère coronaire descendante gauche, le composé a été administré par voie intraveineuse à doses croissantes: 0,5—1—2, 5—5—10 mg/kg, toutes les 30 minutes.

Le nombre de battements d'origine sinusale, nul en témoin, augmente progressivement à partir de la dose de 2,5 mg/kg, 5%+ de battements sinusaux à cette dose, 19% à 5 mg/kg, 6 minutes après l'injection, 41% 20 minutes après l'injection, 25% à 40 minutes et 16% à 60 minutes.

En même temps, le composé est modérément bradycardissant: la fréquence cardiaque passe de 231 à 174 bat/min, soit −25% après la dose de 10 mg/kg, et entraine à cette dose des vomissements brefs.

Ce même chien reçoit, 4 jours après la ligature de la coronaire, des injections I.V. de 5 mcg/kg d'adrénaline qui déclenchent des arythmies. Le nombre des battementes ectopiques déclenchés par l'adrénaline est diminué à partir de la dose de 5 mg/kg I.V. puisque le pourcentage de battements ectopiques par rapport à la fréquence cardiaque totale est de 79% par rapport à 100% en témoin et de 46% 10 minutes après la dose de 10 mg/kg (64% après 40 minutes, 88% après 90 minutes).

Afin de déterminer la distance entre la dose anti-arythmisante et la dose déclenchant des effets toxiques, un chien éveillé reçoit toutes les 45 minutes, par voie intraveineuse, le composé à la dose de 10 mg/kg, 5 fois, plus une dose de 20 mg/kg, soit en dose cumulée 70 mg/kg I.V. Le chien a vomi après 40 mg/

kg et 70 mg/kg, 5 minutes après l'injection; la membrane nictitante a été relâchée durant 10 minutes à 70 mg/kg; on observe une certaine somnolence chez cet animal.

c) Action antiarythmisante chez la souris

Une recherche d'activité anti-arythmisante par voie intrapéritoneale sur le test de Lawson chez la souris (fibrillation ventriculaire (F.V.) par inhalation de chloroforme, réponse en tout ou rien) a donné les résultats suivants (10 souris par dose).

| Dose mg/kg I.P. | % de souris ne présentant pas de F.V. |
|---|---|
| 0 | 0 |
| 5 | 22 |
| 10 | 50 |
| 20 | 70 |

d) Action antiarythmisante chez le cobaye

Le composé est administré par voie intraveineuse à la dose de 10 mg/kg à une série de 10 cobayes albinos de 380 à 495 g, anesthésiés à l'uréthane.

Ils reçoivant, 5 minutes plus tard, une perfusion I.V. du produit arythmisant à la dose de 0,09 mg/min sous un volume de 0,6 ml/min.

Le composé est comparé à la quinidine à la dose de 5 mg/kg I.V. et à une série de témoins.

Les traitements sont tirés au sort.

A — Action sur les arythmies à la K-Strophantine.

Le composé, à la dose de 10 mg/kg I.V. retarde de façon significative l'apparition des accidents de l'E.C.G. dus à la K-Strophantine, sauf celle de la 1ère extra systole.

Il ne modifié pas de façon significative la fréquence cardiaque.

B — Action sur les arythmies à l'aconitine

Le composé, à la dose de 10 mg/kg I.V. retarde de façon significative l'apparition des accidents de l'E.C.G. dus à l'aconitine. Sur ce groupe d'animaux, le composé, à la dose de 10 mg/kg I.V. augmente la fréquence cardiaque de 12 bat/min en moyenne (tachycardie chez 6 animaux/10).

La Quinidine, à la dose de 5 mg/kg I.V., ne retarde significativement que l'apparition de la tachycardie ventriculaire. Ce retard (+30% de moyenne) est significativement inférieur à celui observé avec le composé selon l'invention.

C — Conclusion

Le composé CRL 41 391, administré à la dose de 10 mg/kg I.V., au cobaye anesthésié retarde significativement l'apparition des accidents de l'E.C.G. engendrés par la K-Strophantine et l'aconitine, sans modifier ou en augmentant très modérément la fréquence cardiaque.

Son action vis-à-vis de l'aconitine est supérieure à celle observée après pré-traitement par 5 mg/kg de quinidine.

Il apparait ainsi que le composé se présente comme un produit vasodilateur fémoral possédant des propriétés anti-arythmisantes intéressantes car présentes à dose faible par rapport à la dose toxique.

2) Composé de l'exemple 2 (CRL 41389)

Le composé de l'exemple 2 en solution dans l'eau distillée (pH 5,5 à 6,0), a été administré par voie intrapéritonéale sous un volume de 20 ml/kg chez la souris (mâle, NMRI, C.E.R. Janvier) et de 5 ml/kg chez le rat (mâle, $CD_1$, SPRAGUE DAWLEY, Charles River.

Prétoxicité

— 128 mg/kg (3 souris) sédation et diminution de la fréquence respiratoire pendant 5 minutes. Pas de mortalité.

— 256 mg/kg (6 souris) sédation et diminution de la fréquence respiratoire, convulsions (2/6). Mortalité (1/6), 5 minutes après l'injection.

— 512 mg/kg (6 souris) diminution de la fréquence respiratoire, convulsions (5/6). Mortalité (3/5), 5 minutes après l'injection.

— 1024 mg/kg (6 souris) convulsions, diminution de la fréquence respiratoire. Mortalité (3/3), 4 minutes après l'injection.

Le composé CRL 41 389 provoque une potentialisation modérée des stéréotypies amphétaminiques

chez le rat, une diminution de l'agressivité intergroupes et une aggravation de l'hypothermie réserpinique.

Sur le plan cardiologique le composé diminue les arythmies entraînées par injection I.V. d'adrénaline chez les chiens dont l'artère coronaire a été ligaturée précédemment, dès 2,5 mg/kg I.V.

Le composé est chez l'homme un antidépresseur stimulant.

3) Composé de l'exemple 3 (CRL 41 390)

La toxicité subaigüe du CRL 41 390 est analogue à celle du CRL 41 389.

3 chiens (poids moyen: 14 kg) anesthésiés au nembutal reçoivent le CRL 41 390 par voie intraduodénale, aux doses successives de 0,1—0,5—1—2,5—5—10—20 mg/kg, puis une dose supplémentaire de 10 mg/kg I.V.

On mesure la pression artérielle, la fréquence cardiaque, le débit artériel fémoral, le débit artériel vertébral, les températures rectale et cutanée. On observe la coloration de la peau.

Le CRL 41 390 n'a pas d'effet sur la pression artérielle; il est braydycardisant d'une façon significative à partir de la dose de 2,5 mg/kg I.V. et à la dose de 20 mg/kg par voie intraduodénale. On observe un légère augmentation du débit fémoral à la dose forte (20 mg/kg) surtout chez un chien et une diminution du débit vertébral. Les témpératures rectale et cutanée diminuent modérément.

Les effets de l'isoprénaline sont légèrement diminués sur la pression artérielle diastolique et la fréquence cardiaque: à 10 mcg/kg d'isoprénaline, la pression artérielle diastolique passe après administration du produit de 131 à 44 mmHg/135 à 35 mmHg en témoin, et la fréquence cardiaque passe après produit de 148 à 250 bat/min/ 185 à 272 bat/min en témoin.

L'hypertension à la noradrénaline n'est pas modifiée: à 2 mcg/kg de noradrénaline, la pression artérielle systolique passe après administration du produit de 160 à 299 mmHg/161 à 289 mmHg en témoin.

De plus, le CRL 41 390 est dépourvu d'action anti-arythmisante chez le chien éveillé.

Il apparait que les composés selon l'invention possèdant des propriétiés inattendues par rapport au composé de la technique antérieure qui peut être considéré comme le plus voisin à savoir le composé CRL 41 034.

En effet les composés selon l'invention sont dépourvus d'effet sur la pression artérielle alors que le CRL 41 034 a un effet hypotenseur.

Le composé de l'exemple 1 a donné sous forme de gélule ou de comprimé dosés à 200 mg, 3 à 4 par jour de bons résultats dans le traitement des arythmies cardiaques chez l'homme.

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

**Revendications pour les Etats contractants: BE CH DE FR GB IT LI LU NL SE**

1. Composés de formule

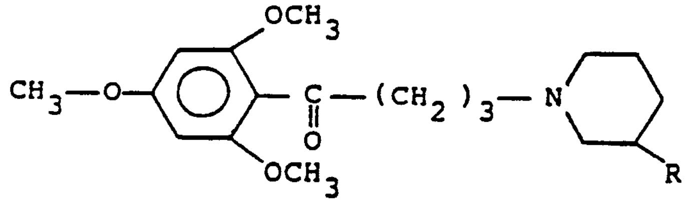

I

dans laquelle R est choisi parmi les groupes $—CH_2OH$, —COOH et $—COOR_1$, $R_1$ étant un groupe alkyle en $C_1$ à $C_4$, et leurs sels pharmaceutiquement acceptables.

2. Le N-[(triméthoxy-2,4,6 benzoyl)-3 propyl] pipéridine-3-méthanol et ses sels pharmaceutiquement acceptables.

3. Procédé de préparation d'un composé selon la revendication 1, caractérisé en ce qu'on effectue une condensation d'un composé de formule

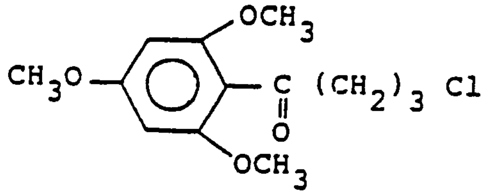

II

avec une pipéridine de formule

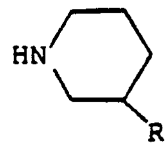

III

R ayant la signification donnée à la revendication 1.

4. Procédé de préparation d'un composé selon la revendication 1, caractérisé en ce qu'on effectue une condensation d'un butyronitrile de formule

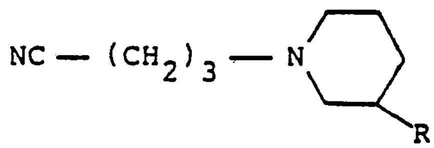

IV

dans laquelle R a la signification donnée à la revendication 1, sur l'éther triméthylique du phloroglucinol, selon une réaction de Houben-Hoesch, en présence d'acide chlorhydrique sans catalyseur, puis on effectue une hydrolyse acide.

5. Composition thérapeutique comprenant à titre de principe actif un composé selon la revendication 1.

6. Composition thérapeutique comprenant à titre de principe actif un composé selon la revendication 2.

**Revendications pour les Etats contractants: AT ES**

1. Procédé de préparation de composés de formule

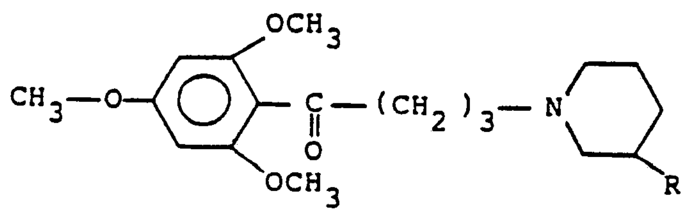

I

dans laquelle R est choisi parmi les groupes $—CH_2OH—$, $—COOH$ et $COOR_1$, $R_1$ étant un groupe alkyle en $C_1$ à $C_4$, et de leurs sels pharmaceutiquement acceptables, caractérisé en ce que l'on effectue une condensation d'un composé de formule

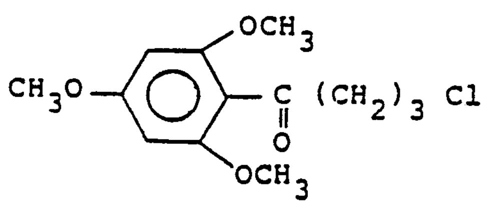

II

avec une pipéridine de formule

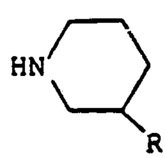

III

R ayant la signification donnée ci-dessus.

2. Procédé de préparation de composés de formule

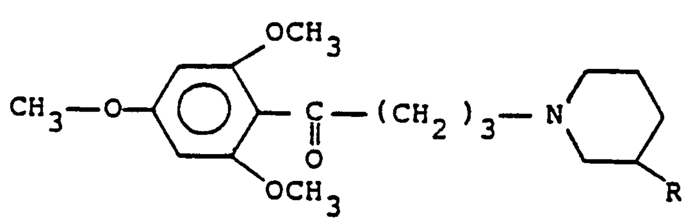

I

dans laquelle R est choisi parmi les groupes —$CH_2OH$—, COOH et —$COOR_1$, $R_1$ étant un groupe alkyle en $C_1$ à $C_4$, et de leurs sels pharmaceutiquement acceptables, caractérisé en ce que l'on effectue une condensation d'un composé de formule

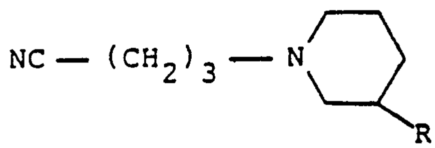

IV

dans laquelle R a la signification donnée ci-dessus, sur l'éther triméthylique de phloroglucinol, selon une réaction de Houben-Hoesch, en présence d'acide chlorhydrique sans catalyseur, puis on effectue une hydrolyse acide.

3. Procédé de préparation d'une composition thérapeutique, caractérisé en ce que l'on met un composé de formule

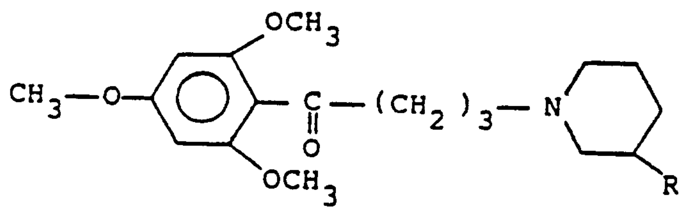

I

dans laquelle R est choisi parmi les groupes —$CH_2OH$—, COOH et $COOR_1$, $R_1$ étant un groupe alkyle en $C_1$ à $C_4$, ou l'un de ses sels pharmaceutiquement acceptables sous une forme pharmaceutiquement acceptable.

4. Procédé de préparation d'une composition thérapeutique, caractérisé en ce que l'on met le N-[(triméthoxy-2,4,6-benzoyl)-3 propyl] pipéridine-3-méthanol ou l'un de ses sels pharmaceutiquement acceptables sous une forme pharmaceutiquement acceptable.

**Revendications pour l'Etat contractant: GR**

1. Composés de formule

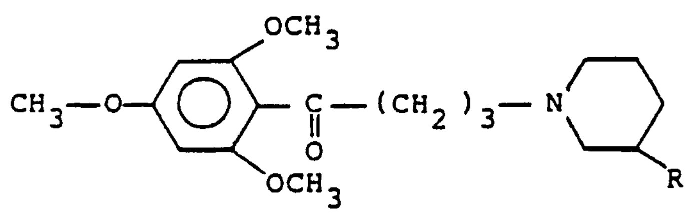

I

dans laquelle R est choisi parmi les groupes —$CH_2OH$—, COOH et —$COOR_1$, $R_1$ étant un groupe alkyle en $C_1$ à $C_4$, et leurs sels pharmaceutiquement acceptables.

2. Le N-[(triméthoxy-2,4,6 benzoyl)-3 propyl] pipéridine-3-méthanol et ses sels pharmaceutiquement acceptables.

3. Procédé de préparation d'un composé selon la revendication 1, caractérisé en ce qu'on effectue une condensation d'un composé de formule

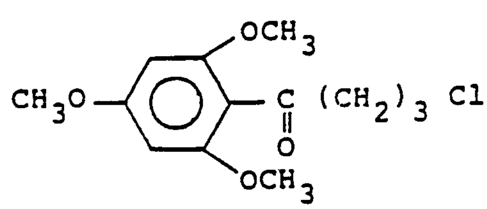

II

avec une pipéridine de formule

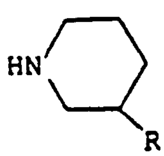

III

R ayant la signification donnée à la revendication 1.

4. Procédé de préparation d'un composé selon la revendication 1, caractérisé en ce qu'on effectue une condensation d'un butyronitrile de formule

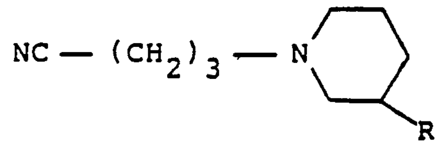

IV

dans laquelle R a la signification donnée à la revendication 1, sur l'éther triméthylique du phloroglucinol, selon une réaction de Houben-Hoesch, en présence d'acide chlorhydrique sans catalyseur, puis on effectue une hydrolyse acide.

5. Procédé de préparation d'une composition thérapeutique, caractérisé en ce que l'on met un composé de formule

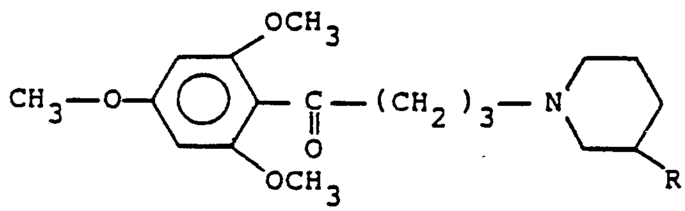

I

dans laquelle R est choisi parmi les groupes $-CH_2OH-$, COOH et $COOR_1$, $R_1$ étant un groupe alkyle en $C_1$ à $C_4$, ou l'un de ses sels pharmaceutiquement acceptables sous une forme pharmaceutiquement acceptable.

6. Procédé de préparation d'une composition thérapeutique, caractérisé en ce que l'on met le N-[(triméthoxy-2,4,6-benzoyl)-3 propyl] pipéridiné-3-méthanol ou l'un de ses sels pharmaceutiquement acceptables sous une forme pharmaceutiquement acceptable.

**Patentansprüche für die Vertragsstaaten: BE CH DE FR GB IT LI LU NL SE**

1. Verbindungen der Formel

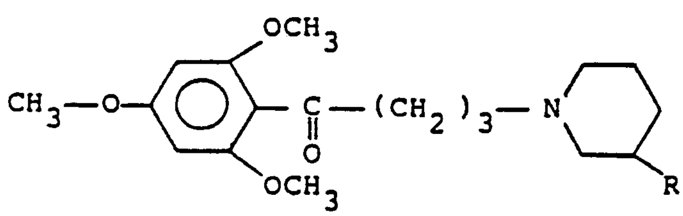

I

worin R aus Gruppen der Formeln $-CH_2OH$, $-COOH$ und $-COOR_1$, worin $R_1$ eine Alkylgruppe mit 1 bis 4 Kohlenstoffatomen bedeutet, ausgewählt ist, und ihre pharmazeutisch annehmbaren Salze.

2. N-[3-(2,4,6-trimethoxybenzoyl)-propyl]-3-hydroxymethylpiperidin und seine pharmazeutisch annehmbaren Salze.

3. Verfahren zur Herstellung einer Verbindung nach Anspruch 1, dadurch gekennzeichnet, daß man eine Verbindung der Formel

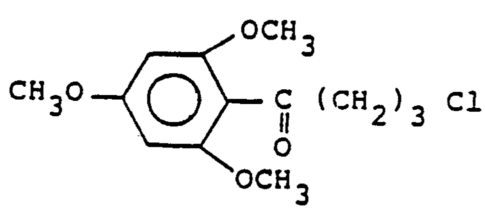

II

mit einem Piperidin der Formel

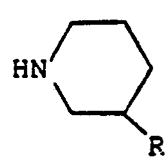

III

worin R die in Anspruch 1 angegebene Bedeutung hat, kondensiert.

4. Verfahren zur Herstellung einer Verbindung nach Anspruch 1, dadurch gekennzeichnet, daß man ein Butyronitril der Formel

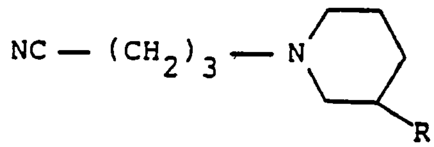

IV

worin R die in Anspruch 1 angegebene Bedeutung hat, nach einer Houben-Hösch-Reaktion mit Phloroglucintrimethylether in Gegenwart von Chlorwasserstoffsäure ohne Katalysator kondensiert und dann eine saure Hydrolyse durchführt.

5. Therapeutische Zusammensetzung, enthaltend als aktiven Wirkstoff eine Verbindung nach Anspruch 1.

6. Therapeutische Zusammensetzung, enthaltend als aktiven Wirkstoff eine Verbindung nach Anspruch 2.

**Patentansprüche für die Vertragsstaaten: AT ES**

1. Verfahren zur Herstellung von Verbindungen der Formel

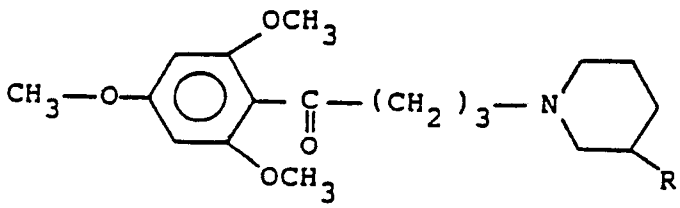

I

worin R aus Gruppen der Formeln $—CH_2OH—$, $—COOH$ und $COOR_1$, worin $R_1$ eine Alkylgruppe mit 1 bis 4 Kohlenstoffatomen bedeutet, ausgewählt ist, und ihren pharmazeutisch annehmbaren Salzen, dadurch gekennzeichnet, daß man eine Verbindung der Formel

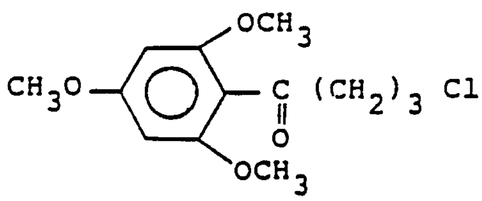

II

mit einem Piperidin der Formel

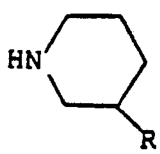

III

worin R die oben angegebene Bedeutung hat, kondensiert.

2. Verfahren zur Herstellung von Verbindungen der Formel

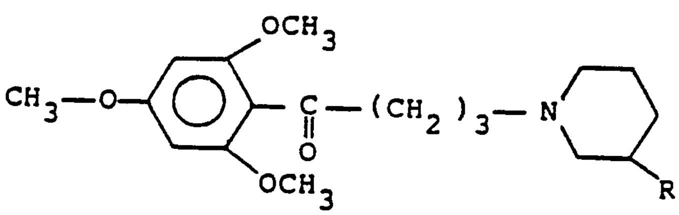

I

worin R aus Gruppen der Formeln $—CH_2OH$, $—COOH$ und $COOR_1$, worin $R_1$ eine Alkylgruppe mit 1 bis 4 Kohlenstoffatomen bedeutet, ausgewählt ist und ihren pharmazeutisch annehmbaren Salzen, dadurch gekennzeichnet, daß man ein Butyronitril der Formel

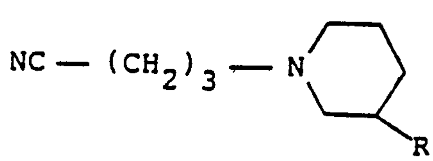

IV

worin R die oben angegebene Bedeutung hat, nach einer Houben-Hösch-Reaktion mit Phloroglucintrimethylether in Gegenwart von Chlorwasserstoffsäure ohne Katalysator kondensiert und dann eine saure Hydrolyse durchführt.

3. Verfahren zur Herstellung einer therapeutischen Zusammensetzung, dadurch gekennzeichnet, daß man eine Verbindung der Formel

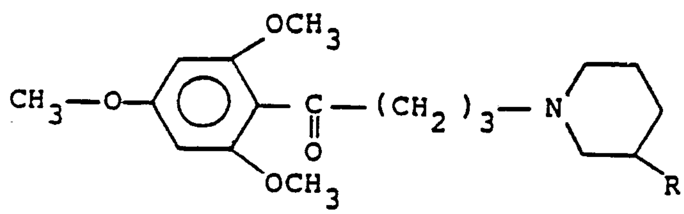

I

worin R aus Gruppen der Formeln $-CH_2OH$, $-COOH$ und $COOR_1$, worin $R_1$ eine Alkylgruppe mit 1 bis 4 Kohlenstoffatomen bedeutet, ausgewählt ist, oder eines ihrer pharmazeutisch annehmbaren Salze in eine pharmazeutisch annehmbare Form bringt.

4. Verfahren zur Herstellung einer therapeutischen Zusammensetzung, dadurch gekennzeichnet, daß man N-[3-(2,4,6-Trimethoxybenzoyl)-propyl]-3-hydroxymethylpiperidin oder eines seiner pharmazeutisch annehmbaren Salze in eine pharmazeutisch annehmbare Form bringt.

**Patentansprüche für den Vertragsstaat: GR**

1. Verbindungen der Formel

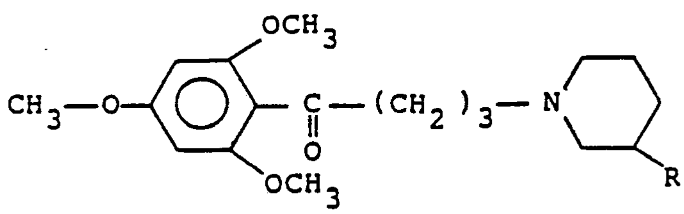

I

worin R aus Gruppen der Formeln $-CH_2OH$, $-COOH$ und $-COOR_1$, worin $R_1$ eine Alkylgruppe mit 1 bis 4 Kohlenstoffatomen bedeutet, ausgewählt ist, und ihre pharmazeutisch annehmbaren Salze.

2. N-[3-(2,4,6-Trimethoxybenzoyl)-propyl]-3-hydroxymethylpiperidin und seine pharmazeutisch annehmbaren Salze.

3. Verfahren zur Herstellung einer Verbindung nach Anspruch 1, dadurch gekennzeichnet, daß man eine Verbindung der Formel

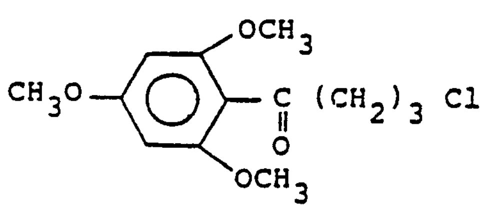

II

mit einem Piperidin der Formel

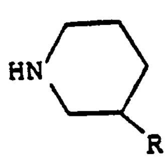

III

worin R die Anspruch 1 angegebene Bedeutung hat, kondensiert.

4. Verfahren zur Herstellung einer Verbindung nach Anspruch 1, dadurch gekennzeichnet, daß man ein Butyronitril der Formel

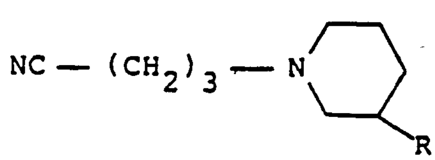

IV

worin R die in Anspruch 1 angegebene Bedeutung hat, nach einer Houben-Hösch-Reaktion mit Phloroglucintrimethylether in Gegenwart von Chlorwasserstoffsäure ohne Katalysator kondensiert und dann eine saure Hydrolyse durchführt.

5. Verfahren zur Herstellung einer therapeutischen Zusammensetzung, dadurch gekennzeichnet, daß man eine Verbindung der Formel

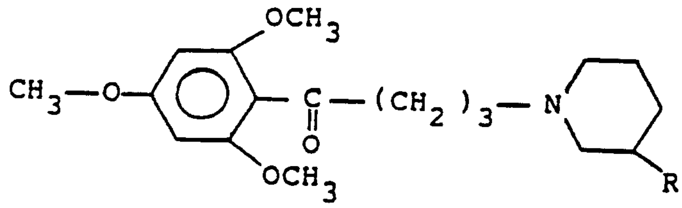

I

worin R aus Gruppen der Formeln $-CH_2OH$, $-COOH$ und $COOR_1$, worin $R_1$ eine Alkylgruppe mit 1 bis 4 Kohlenstoffatomen bedeutet, ausgewählt ist, oder eines ihrer pharmazeutisch annehmbaren Salze in eine pharmazeutisch annehmbare Form bringt.

6. Verfahren zur Herstellung einer therapeutischen Zusammensetzung, dadurch gekennzeichnet, daß man N-[3-(2,4,6-Trimethoxybenzoyl)-propyl]-3-hydroxymethylpiperidin oder eines seiner pharmazeutisch annehmbaren Salze in eine pharmazeutisch annehmbare Form bringt.

**Claims for the Contracting States: BE CH DE FR GB IT LI LU NL SE**

1. Compounds having the formula

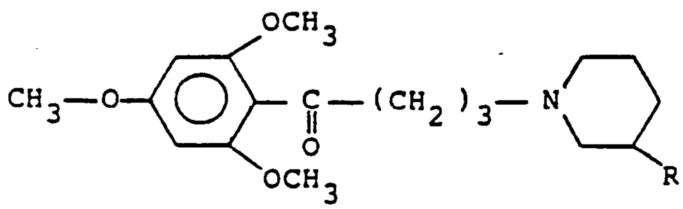

I

in which R is selected from the groups $-CH_2OH$, $-COOH$ and $-COOR_1$, $R_1$ being a $C_1$ to $C_4$ alkyl group, and their pharmaceutically acceptable salts.

2. N-[(2,4,6-trimethoxy benzoyl)-3 propyl] piperidine-3-methanol and its pharmaceutically acceptable salts.

3. A process for the preparation of a compound according to claim 1, characterised in that a compound having the formula

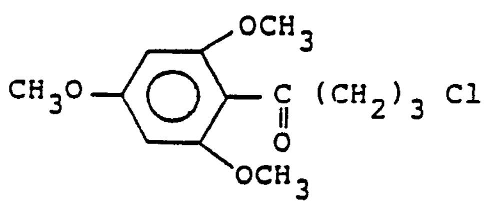

II

is condensed with a piperidine having the formula

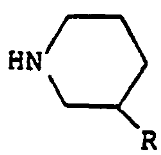

III

wherein R is as given in claim 1.

4. A process for the preparation of a compound in accordance with claim 1, characterised in that a butyronitrile having the formula

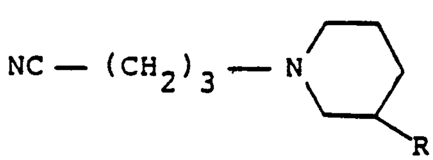

IV

in which R is as given in claim 1, is condensed on phloroglucinol trimethyl ether, in accordance with a Houben-Hoesch reaction, in the presence of hydrochloric acid without a catalyst, then acid hydrolysis is carried out.

5. A therapeutic composition comprising a compound according to claim 1 as the active principle.

6. A therapeutic composition comprising a compound according to claim 2 as the active principle.

**Claims for the Contracting States: AT ES**

1. A process for the preparation of compounds having the formula

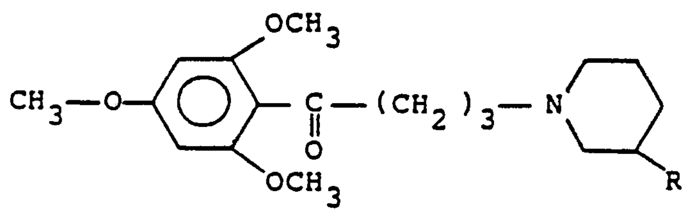

I

in which R is selected from the groups $—CH_2OH$, $—COOH$ and $COOR_1$, $R_1$ being a $C_1$ to $C_4$ alkyl group, and their pharmaceutically acceptable salts, characterised in that a compound having the formula

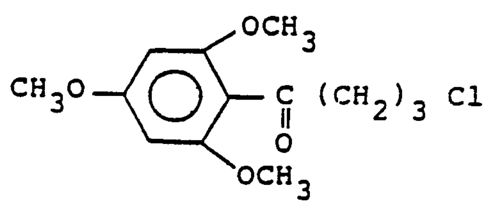

II

is condensed with a piperidine having the formula

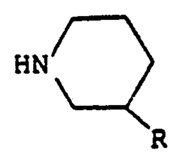

III

wherein R is as given above.

2. A process for the preparation of compounds having the formula

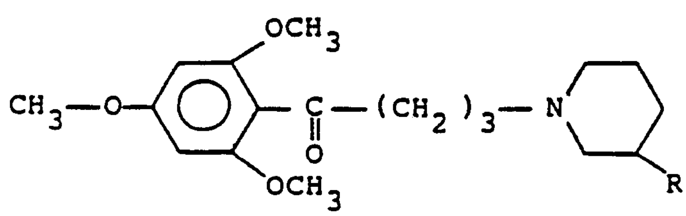

I

in which R is selected from the groups $—CH_2OH$, $—COOH$ and $—COOR_1$, $R_1$ being a $C_1$ to $C_4$ alkyl group, and their pharmaceutically acceptable salts, characterised in that a butyronitrile having the formula

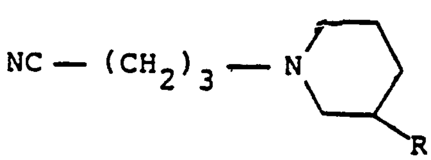

IV

in which R is as given above, is condensed on phloroglucinol trimethyl ether, in accordance with a Houben-Hoesch reaction, in the presence of hydrochloric acid without a catalyst, then acid hydrolysis is carried out.

3. A process for the preparation of a therapeutic composition, characterised in that a compound having the formula

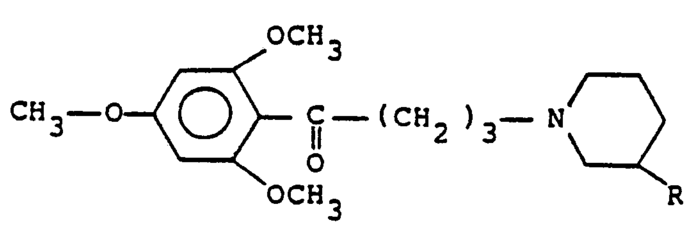

I

in which R is selected from the groups $—CH_2OH$, $—COOH$ and $COOR_1$, $R_1$ being a $C_1$ to $C_4$ alkyl group, or one of its pharmaceutically acceptable salts, is put into a pharmaceutically acceptable form.

4. Process for the preparation of a therapeutic composition, characterised in that the N-[(2,4,6-trimethoxy benzoyl)-3-propyl] piperidine-3-methanol or one of its pharmaceutically acceptable salts is put into a pharmaceutically acceptable form.

**Claims for the Contracting State: GR**

1. Compounds having the formula

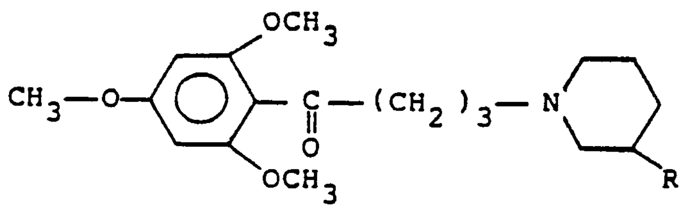

I

in which R is selected from the groups $—CH_2OH$, $—COOH$ and $—COOR_1$, $R_1$ being a $C_1$ to $C_4$ alkyl group, and their pharmaceutically acceptable salts.

2. N-[(2,4,6-trimethoxy benzoyl)-3 propyl] piperidine-3-methanol and its pharmaceutically acceptable salts.

3. A process for the preparation of a compound in accordance with claim 1, characterised in that a compound having the formula

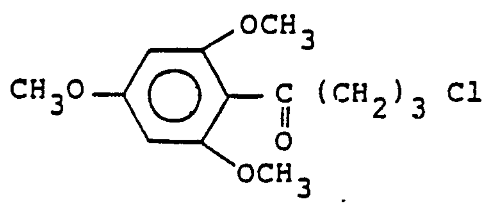

II

is condensed with a piperidine having the formula

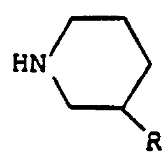

III

wherein R is as given above.

4. A process for the preparation of a compound in accordance with claim 1, characterised in that a butyronitrile having the formula

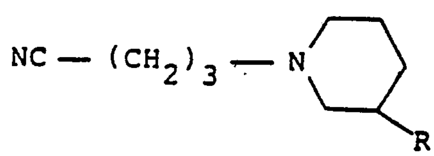

IV

in which R is as given in claim 1, is condensed on phloroglucinol trimethyl ether, in accordance with a Houben-Hoesch reaction, in the presence of hydrochloric acid without a catalyst, then acid hydrolysis is carried out.

5. A process for the preparation of a therapeutic composition, characterised in that a compound having the formula

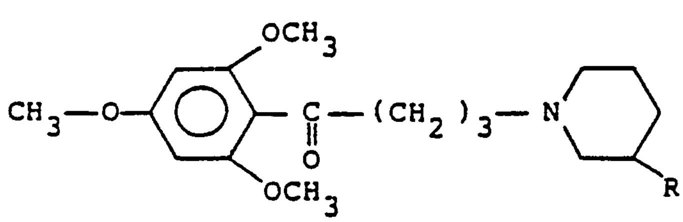

I

in which R is selected from the groups —$CH_2OH$, —COOH and $COOR_1$, $R_1$ being a $C_1$ to $C_4$ alkyl group, or one of its pharmaceutically acceptable salts, is put into pharmaceutically acceptable form.

6. A process for the preparation of a therapeutic composition, characterised in that the N-[(2,4,6-trimethoxy benzoyl)-3-propyl] piperidine-3-methanol, or one of its pharmaceutically acceptable salts, is put into pharmaceutically acceptable form.